# EUROPEAN PATENT APPLICATION

(11) **EP 1 285 624 A2**
(43) Date of publication of application: **26.02.2003**
(21) Application number: 02352009.1
(22) Date of filing: 21.03.2002
(51) Int. Cl.: A61B 3/12, A61B 3/135

(54) **Ophtalmic examination and treatment system**

(30) Priority: 17.08.2001 JP 2001006210
(71) Applicant: Makino Ophtalmic Instrument Co., Ltd., Tokyo 113-0033 (JP)
(72) Inventor: Makino, Toshimi, Tokyo (JP)
(74) Representative: Morelle, Guy Georges Alain

(57) **Abstract**

An object of the present invention is to provide an ophthalmic examination and treatment system capable of improving the usability of the imaging device mounted on an ophthalmic medical instrument, simplifying the relationship with associated devices such as the display device and recording device thereof, and improving the overall user-friendliness thereof. The ophthalmic examination and treatment system of the present invention is characterized in that a wireless imaging device (wireless CCD camera) is employed as the imaging device for various ophthalmic medical instruments, and the transmission/reception of information data with the display device and recording device is conducted by using wireless communication means.

## Description

The present invention relates to a medical instrument, an ophthalmic medical instrument to be used in ophthalmic diagnosis, and particularly to an ophthalmic examination and treatment system equipped with the likes of an imaging device and a display device for displaying the captured images thereof.

As ophthalmic medical instruments employed during ophthalmic diagnosis, for example, slit lamps, fundoscopy devices, indirect ophthalmoscopes, and ophthalmic operation microscopes used in ophthalmic surgery are known, as are operation microscopes used in brain surgery.

With the foregoing medical instruments, an imaging device is built therein permanently or mounted as an option, and this imaging device is used for imaging the condition of the affected area and the state of treatment, and displaying this on a monitor screen, which is a display device provided separately.

Meanwhile, although a CCD camera is employed as the aforementioned imaging device in this type of ophthalmic medical instrument, there is a problem in that a fixed-line system where the camera and the display device are generally connected with a cable is typically used, and inconveniences arise with respect to the workability, mobility, installation, and so on.

In other words, upon attaching the aforementioned imaging device to a part of the ophthalmic medical instrument which is the primary instrument, it is necessary to provide a cable for transmitting the image from the imaging device to the likes of a display device. In addition, even upon installing the imaging device at a position in the vicinity of the object to be imaged, since this location is also an important section for the primary instrument, the installation position is easily restricted and cases arise where adequate imaging becomes difficult.

Moreover, although this is not be much of a problem when the aforementioned ophthalmic medical instrument is installed and fixed in an ophthalmic examination room or operating room, a problem arises when using this as a transportable instrument in a traveling clinic. Particularly, since each ophthalmic medical instrument has an integral portion with a primary function, such primary portions also have numerous power cables and transmission cables. Thus, if there are additional cables for the imaging device, the handling thereof becomes complicated and is sometimes a hindrance in the transportation thereof.

Therefore, measures are being taken whereby the imaging device and display device are removed such that only the primary portion of the ophthalmic medical instrument is transported and used. Nevertheless, there are cases where it is preferable to display or record the state of treatment during such transportation, and it is desired that some type of countermeasure be taken.

Further, as locations for using the aforementioned ophthalmic medical instruments, there are places where the wiring of information transmission cables is not possible, and countermeasures for such a case are also necessary.

The present invention was devised in view of the foregoing circumstances, and an object thereof is to provide an ophthalmic examination and treatment system capable of improving the usability of the imaging device mounted on an ophthalmic medical instrument, simplifying the relationship with the likes of the display device and recording device thereof, and improving the overall user-friendliness thereof.

In order to achieve the foregoing object, the ophthalmic examination and treatment system according to the present invention (invention claimed in claim 1) comprises: an ophthalmic medical instrument used in ophthalmic diagnosis; an imaging device mounted on the ophthalmic medical instrument; and a display device for displaying images captured with the imaging device; wherein a wireless imaging device is used as the imaging device, and the transmission/reception of information data with the display device is conducted with wireless communication means.

The ophthalmic examination and treatment system of the present invention (invention claimed in claim 2) according to claim 1 further comprises a recording device for recording the image displayed on the display device.

The ophthalmic examination and treatment system of the present invention (invention claimed in claims 3 to 5) according to claim 1 or claim 2 is characterized in that the ophthalmic examination instrument is an operation microscope, a slit lamp, or a fundoscopy device.

The ophthalmic examination and treatment system of the present invention (invention claimed in claim 6) according to any one of claims 1 to 5 is characterized in that the imaging device is a wireless CCD camera.

According to the present invention, by employing a wireless imaging device to be mounted on a suitable position on the ophthalmic medical instrument, not only is it possible to overcome problems such as the installation position thereof being restricted, but the imaging device will also be capable of imaging necessary sites as the objects of imaging, thus exhibiting its functions to the fullest.

Moreover, since a wireless imaging device is employed, cables for connection with the display device and recording device are no longer required, and since the primary instrument and the wiring thereof will not be a hindrance, a user-friendly imaging system can be constituted. Further, the ophthalmic medical instrument, which is the primary instrument, can be transported freely.
Fig. 1 is an explanatory diagram for showing an embodiment of the ophthalmic examination and treatment system according to the present invention, and explaining the overall description thereof;
Fig. 2 is a schematic perspective view of a slit lamp as an example of the ophthalmic medical instrument to which the ophthalmic examination and treatment system according to the present invention is applied;
Fig. 3 is an explanatory view for explaining the outline of the ophthalmic examination and treatment system employing the slit lamp illustrated in Fig. 2; and
Fig. 4 is a schematic perspective view of an operation microscope as an example of the ophthalmic medical instrument to which the ophthalmic examination and treatment system according to the present invention is applied.

The present invention will now be described in detail by reference to the embodiments illustrated in the drawings.

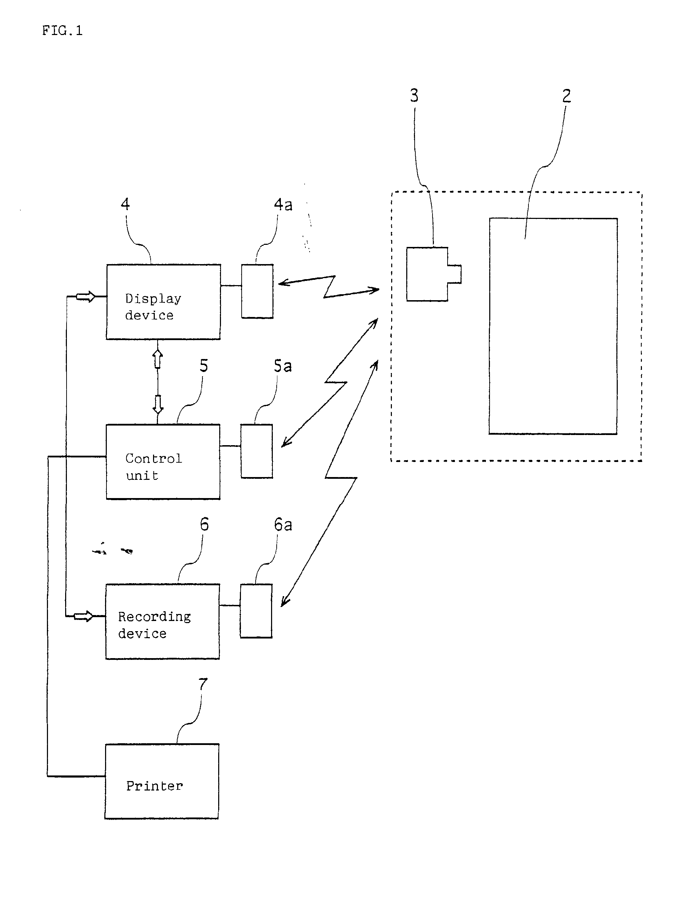

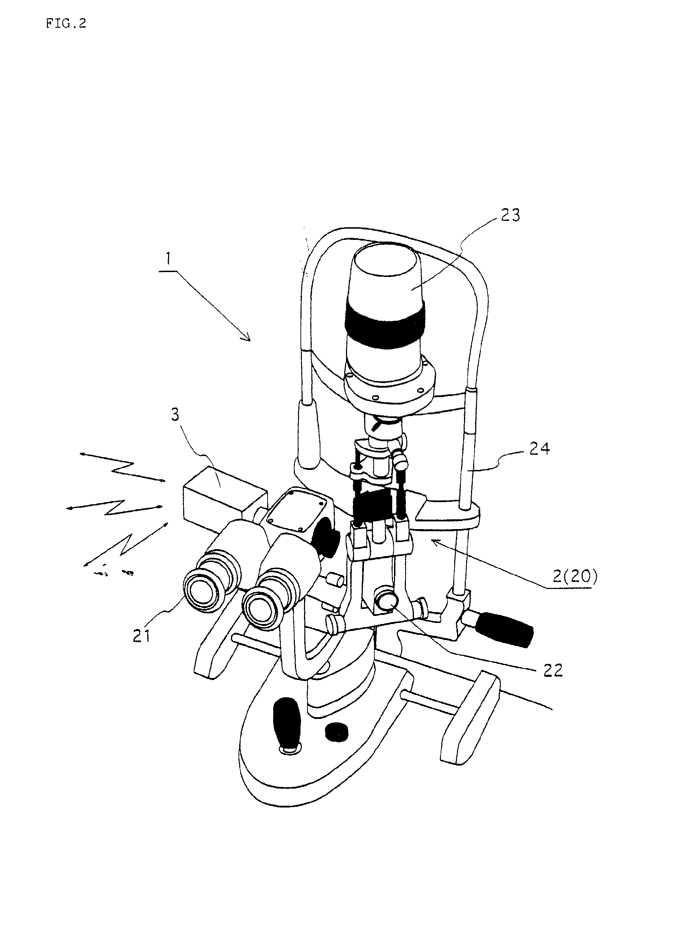

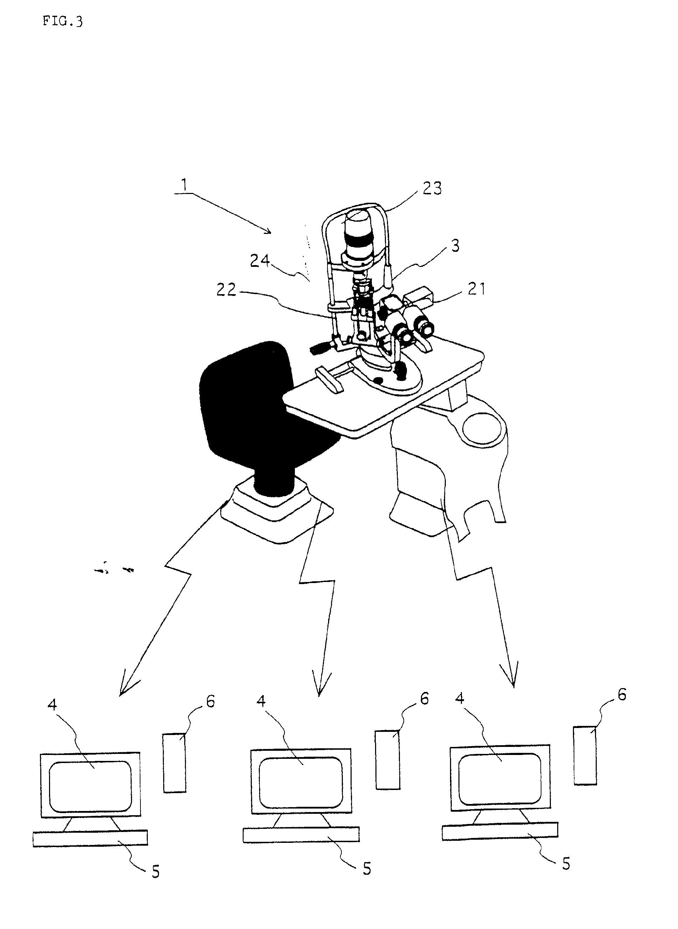

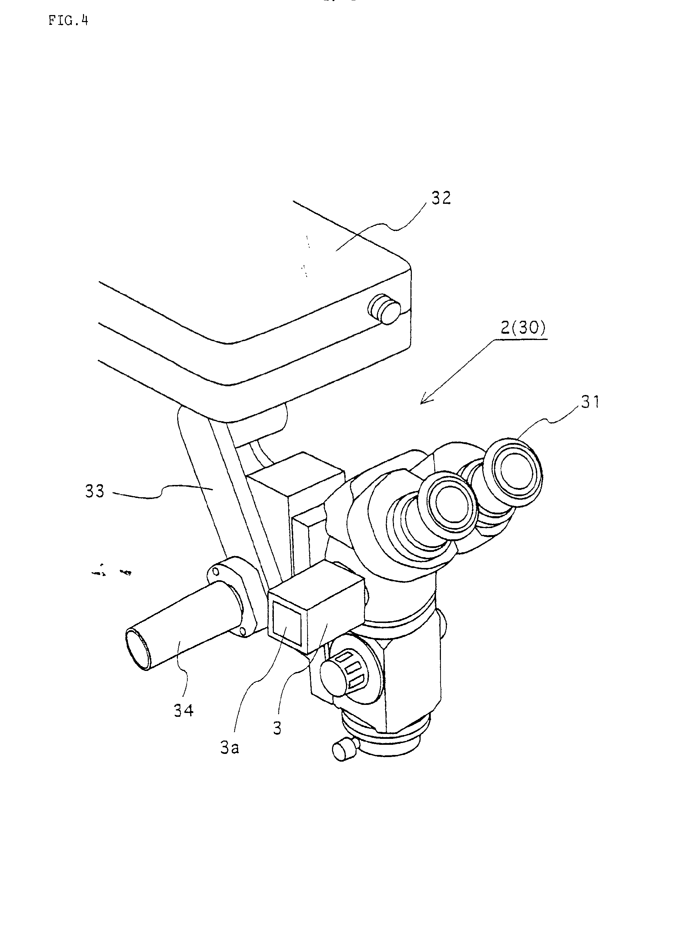

In the drawings, Fig. 1 is an explanatory diagram for showing one embodiment of the ophthalmic examination and treatment system according to the present invention, and explaining the overall description thereof. Fig. 2 is a schematic perspective view of a slit lamp as an example of the ophthalmic medical instrument to which the ophthalmic examination and treatment system according to the present invention is applied. Fig. 3 is an explanatory view for explaining the outline of the ophthalmic examination and treatment system employing the slit lamp illustrated in Fig. 2. Fig. 4 is a schematic perspective view of an operation microscope as an example of the ophthalmic medical instrument to which the ophthalmic examination and treatment system according to the present invention is applied.

The overall ophthalmic examination and treatment system is represented with reference numeral 1, and comprises an ophthalmic medical instrument 2 such as a slit lamp 20, an operation microscope 30 or a fundoscopy device, all described later, used in ophthalmic diagnosis; an imaging device 3 mounted on this ophthalmic medical instrument 2; and a display device 4 for displaying the images captured with this imaging device 3.

The present invention is characterized in that a wireless imaging device 3 (wireless CCD camera) is employed as the imaging device, and the transmission/reception of information data with the display device 4 is conducted with wireless communication means.

Here, reference numeral 5 in the diagram represents a control unit used when manually operating the aforementioned display device 4 and also the imaging device 3. Reference numeral 6 is a recording device for directly recording images obtained with the imaging device 3, or indirectly recording, via the display device 4, images displayed on the display device 4. Each of these devices 4, 5 and 6 is respectively provided with a transmission/reception unit 4a, 5a and 6a for transmitting and receiving signals to and from the transmission/reception unit of the imaging device 3 (provided to the rear end portion of the camera, for example). Further, the present invention may also be structured to output the displayed image from a printer 7 as necessary.

Here, a slit lamp 20 as illustrated in Fig. 2 and Fig. 3, an operation microscope 30 as illustrated in Fig. 4, or a fundoscopy device may be used as the aforementioned ophthalmic medical instrument 2; that is, any instrument conventionally known to be used for ophthalmic diagnosis may be employed.

According to the ophthalmic examination and treatment system 1 in which the imaging device 3 is mounted on the ophthalmic medical instrument 2 as structured above, by employing a wireless imaging device 3 (wireless CCD camera) to be mounted on a suitable position on the ophthalmic medical instrument 2, not only is it possible to overcome problems such as the installation position thereof being restricted, but the imaging device will also be capable of imaging necessary sites as the objects of imaging, thus exhibiting its functions to the fullest.

Moreover, since a wireless imaging device 3 is employed, cables for connection with the display device 4 and recording device 6 are no longer required, and since the primary instrument (ophthalmic medical instrument) 2 and the wiring thereof will not be a hindrance, a user-friendly imaging system can be constituted. Further, the ophthalmic medical instrument 2, which is the primary instrument, can be transported freely. When transporting the primary instrument 2, only the imaging device 3 needs to be mounted, and there is an advantage in that the display device 4 and recording device 6 may be located at the place of permanent installation, and necessary imaging may be conducted with certainty.

According to the foregoing structure, it is possible to obtain an ophthalmic examination and treatment system 1 capable improving the usability of the imaging device 3 mounted on the ophthalmic medical instrument 2, simplifying the relationship with the likes of the display device 4 and recording device 6 thereof, and improving the overall user-friendliness thereof.

Here, Fig. 2 and Fig. 3 illustrate examples of applying the present invention to the slit lamp 20, and this slit lamp 20 comprises an eyepiece tube 21, a beam splitter 22, a loading portion 23 for various internal filters, and a stand 24 onto which the above components are mounted. Also, the imaging device 3 is mounted on a suitable position.

The wireless CCD camera, which is the aforementioned imaging device 3, is structured such that it is capable of sending image signals to a plurality of display devices 4 (three devices in this example) provided at suitable locations separate from the position to which the ophthalmic medical instrument 2 is disposed, and suitably displaying such images.

The slit lamp 20 as the ophthalmic medical instrument structured as above may be moved to any position, and is capable of transmitting images to the display devices 4 provided at separate locations via wireless communication in a simple and accurate manner.

Fig. 4 depicts an example where the imaging device 3 consisting of a wireless CCD camera is mounted on the operation microscope 30 used when performing ophthalmic surgery, thus enabling the transmission of images to the display devices 4 provided at separate locations via wireless communication in a simple and accurate manner even during such surgery.

Here, reference numeral 31 is a variable-power microscope, 32 is a spare lamp unit, 33 is a stand, and 34 is a grip portion.

Needless to say, the aforementioned effects and advantages are also yielded when applying the present invention to this type of variable-power microscope 30.

Further, the present invention is not limited to the structures explained in the foregoing embodiments, and it goes without saying that the shape and structure of the respective components may be suitably modified or changed. For example, in the aforementioned embodiments, examples are given in which the present invention was employed in a slit lamp 20 and an operation microscope 30, but the present invention is not limited thereto, and, needless to say, appropriate portions may be arbitrarily modified.

As described above, according to the ophthalmic examination and treatment system according to the present invention, by employing a wireless imaging device to be mounted on a suitable position on the ophthalmic medical instruments, not only is it possible to overcome problems such as the installation position thereof being restricted, but the imaging device will also be capable of imaging necessary sites as the objects of imaging, thus exhibiting its functions to the fullest.

Moreover, since a wireless imaging device is employed, cables for connection with the display device and recording device are no longer required, and since the primary instrument and the wiring thereof will not be a hindrance, a user-friendly imaging system can be constituted. Further, since a wireless system is adopted, the primary instrument can be operated easily, the weight thereof can be lightened, and this is convenient for transportation.

When transporting the ophthalmic medical instrument, which is the primary instrument, only the imaging device needs to be mounted, and there is an advantage in that the display device and recording device may be located at the place of permanent installation, and necessary imaging and image transmission may be conducted with ease.

## Claims

1. An ophthalmic examination and treatment system comprising: an ophthalmic medical instrument used in ophthalmic examination or treatment; an imaging device mounted on said ophthalmic medical instrument; and a display device for displaying images captured by said imaging device;
**characterized in that** a wireless imaging device is used as said imaging device, and the transmission/reception of information data with said display device is conducted with wireless communication means.

2. An ophthalmic examination and treatment system according to claim 1, further comprising a recording device for recording the image displayed on said display device.

3. An ophthalmic examination and treatment system according to claim 1 or claim 2, **characterized in that** said ophthalmic examination instrument is an operation microscope.

4. An ophthalmic examination and treatment system according to claim 1 or claim 2, **characterized in that** said ophthalmic examination instrument is a slit lamp.

5. An ophthalmic examination and treatment system according to claim 1 or claim 2, **characterized in that** said ophthalmic examination instrument is a fundoscopy device.

6. An ophthalmic examination and treatment system according to any one of claims 1 to 5, **characterized in that** said imaging device is a wireless CCD camera.
